(19) **Europäisches Patentamt European Patent Office Office européen des brevets**

(11) **EP 4 648 735 B1**

(12) **EUROPEAN PATENT SPECIFICATION**

(45) Date of publication and mention of the grant of the patent:
**11.03.2026 Bulletin 2026/11**

(21) Application number: **25719288.0**

(22) Date of filing: **04.04.2025**

(51) International Patent Classification (IPC):
*A61K 8/02* *(2006.01)*  *A61K 8/26* *(2006.01)*
*A61K 8/35* *(2006.01)*  *A61K 8/37* *(2006.01)*
*A61K 8/40* *(2006.01)*  *A61Q 17/04* *(2006.01)*

(52) Cooperative Patent Classification (CPC):
**A61Q 17/04; A61K 8/0279; A61K 8/26; A61K 8/35; A61K 8/37**

(86) International application number:
**PCT/EP2025/059381**

(87) International publication number:
**WO 2025/210279 (09.10.2025 Gazette 2025/41)**

(54) **SUNSCREEN COMPOSITION WITH SPF BOOSTERS**

SONNENSCHUTZZUSAMMENSETZUNG MIT SPF-BOOSTERN

COMPOSITION DE PRODUIT DE PROTECTION SOLAIRE AVEC AMPLIFICATEURS DE FPS

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **05.04.2024 IT 202400007597**
**05.04.2024 IT 202400007600**
**22.07.2024 IT 202400016909**
**22.07.2024 IT 202400016924**

(43) Date of publication of application:
**19.11.2025 Bulletin 2025/47**

(73) Proprietor: **Saes Getters S.p.A.**
**20045 Lainate (MI) (IT)**

(72) Inventors:
• **VACCA, Paolo**
**20146 Milano MI (IT)**
• **MACCHI, Giorgio**
**21013 Gallarate VA (IT)**
• **FIDECKA, Katarzyna**
**22063 Cantu CO (IT)**
• **ZILIO, Stefano**
**20010 Bareggio MI (IT)**
• **RIVA, Miriam**
**22074 Lomazzo CO (IT)**
• **PIROLA, Simona**
**20061 Carugate MI (IT)**
• **DE SIMONE, Agnello**
**21047 Saronno VA (IT)**

(74) Representative: **Novagraaf Group**
**Chemin de l'Echo 3**
**1213 Onex / Geneva (CH)**

(56) References cited:
**WO-A1-2017/112982 WO-A1-2022/081942**
**WO-A1-2022/185348 CA-A1- 3 032 422**
**US-A1- 2005 276 761**

**Description**

**FIELD OF INVENTION**

The present invention relates generally to sunscreen compositions for protecting damage from ultraviolet (UV) rays. More specifically, the invention is direct to a formulation with SPF booster based on microporous particles having a specific particle size distribution (PSD).

**DESCRIPTION**

The negative effects of exposure to ultraviolet (UV) light having a wavelength of from about 200 nm to about 400 nm, are generally recognized. The prolonged non-shielded exposure to solar radiation causes adverse health consequences, such as the immediately appearing of painful sunburns, and long-term damage which can lead to serious conditions such as skin cancer.

UV light also contributes to aging by causing free radicals to form in the skin. Free radicals include, for example, singlet oxygen, hydroxyl radical, the superoxide anion, nitric oxide and hydrogen radicals. Free radicals attack DNA, membrane lipids and proteins, generating carbon radicals. These in turn react with oxygen to produce a peroxyl radical that can attack adjacent fatty acids to generate new carbon radicals. This cascade leads to a chain reaction producing lipid peroxidation products. Damage to the cell membrane results in loss of cell permeability, increased intercellular ionic concentration, and decreased ability to excrete or detoxify waste products.

In order to reduce the amount of solar UV radiation received by the skin during exposure to the sun radiation, different sunscreen compositions can be used.

A sun care composition may contain inorganic UV filters, also called physical filters, and/or chemical UV filters which are organic molecules. The inorganic UV filters interact with UV light by two mechanisms: absorption and reflection/scattering, while the organic filters typically contain aromatic carbon and/or other electron-dense bonds that are responsible for absorbing light in the UV ranges of the solar spectrum.

The higher the amount of UV filters, the greater the degree of UV protection, however, it has recently become evident that too high concentration of both inorganic and organic UV filters, not only compromise the sun care formulation aesthetics, but it also brings unfavorable safety impacts on both human health and the environment.

Therefore, a key challenge that arose in this field is represented by the reduction of the quantities of UV filters used in sunscreen formulations while still ensuring high sun protection efficacy.

According to that, an effective strategy to solve this problem involves the use of a sun protection factor (SPF) booster into sunscreen formulations. SPF boosters are defined as compounds, safe for both human use and the environment, which are not recognized as sunscreen actives, but work to increase the SPF of the composition in which they are incorporated. According to some specific regulations (i.e. European Cosmetics Regulation 1223/2009), SPF formulations have to provide SPF protection consisting in UVA protection factor (UVAPF) at least on third of SPF with a critical wavelength of 370 nm or more. This is needed to reduce the UVA efficiency in penetrating the dermis and in promoting photosensitizing reaction able to generate harmful reactive oxygen species.

Typical sunscreen formulations are emulsions, serums, creams, or gels containing, sprays, in addition to chemical and/or physical UV filters, many other compounds such as emulsifiers, solubilizers, stabilizers, preservatives and also SPF boosters; all of which influence the protectiveness of the sunscreen, the activity of the SPF Booster and the and cosmetic appeal.

As reported in WO2017112982 and WO2021102873 a widely used inorganic SPF booster is represented by hydrophobic silica particles. Also, US9265715 claims for example the use of silica particles in combination with nylon and barium sulfate particles in order to have a boosting effect on sunscreen composition. A further example is represented by US20210330571 in which hydrophobic fine particulate of titanium oxide or zinc oxide, ensure a boosting effect to the final composition. Alternatively, WO2022081942 describes an organic compound, specifically xanthommatin, as SPF booster in combination with a chemical UV filter.

Another typical approach in the field of SPF booster is then represented by the use of particles characterized by specific dimensions which overlap the range of the UV light (i.e. about 200-400 nm), in order to exploit the UV scattering mechanism, as reported in US10485745.

However, some of the most cited classes of compounds such as silica has been progressively reduced due to their toxicity for human and environment. While inorganic compounds as titanium oxide and zinc oxide or organic compounds as the xanthommatin above reported, cannot be considered an SPF booster since they act as a real UV filter (the so-called SPF-doping effect).

Thus, object of the present invention is the development of a sunscreen composition characterized by the presence of a new versatile SPF booster.

In particular, the inventors found that in a sunscreen composition, comprising at least one UV filter, a surprisingly improvement of the booster effect on both UVB and UVA values can be obtained when the SPF booster is based on microporous particles having a particle size distribution (PSD) comprised between 100 nm and 10 $\mu$m, specifically divided into three fractions.

Said fractions are defined as a first fraction from 100 nm to 400 nm, a second fraction from 400 nm to 1 $\mu$m, and a third fraction from 1 to 10 $\mu$m, characterized by a ratio between the amount of the second fraction and the sum of the first and third fractions comprised between 0.1 and 5.

The zeolites particles size distribution (PSD) is measured using a state-of-the-art laser diffraction instrument. The powder is dispersed in DI water using an US bath up to a suitable concentration, to generate the optimum obscuration signal in the instrument. The slurry is recirculated from a container to the detector and the particle size distribution is measured. Particularly, the cumulative number distribution is evaluated.

The diameter fractions are calculated starting from the cumulative curve (e.g. the 0.1 - 0.4um fraction is the cumulative curve value in this range).All the SPF boosters of the present invention do not show a sunscreen activity as a single component, but act as an efficient SPF booster and UVA-PF booster when added in a formulation comprising UV filters; thus avoiding the SPF doper effect according to the international safety regulation.

According to the invention, the amount of SPF booster particles is comprised between 0.1 and 5% wt with respect to the total weight of the composition, while the amount of the UV filter is comprised 1.0 and 35%wt with respect to the total weight of the composition.

In a preferred embodiment, specifically when an organic UV filter is employed, the amount of said compound is comprised between 1 and 15% wt with respect to the total amount of the composition, while the amount is preferably comprised between 1 and 25%wt with respect to the total amount of the composition, when said filter is an inorganic compound.

Indeed, the UV filters employed in the sunscreen composition described herein can be an organic molecule, also defined as chemical filter, an inorganic compound, also defined as physical filter or mixture thereof.

Said organic molecule is selected in a group consisting of Diethylamino Hydroxybenzoyl Hexyl Benzoate, Ethylhexyl Salicylate, Bis-Ethylhexyloxyphenol Methoxyphenyl Triazine, Ethylhexyl Triazone, Phenylbenzimidazole Sulfonic Acid, Octocrylene, Homosalate, Butyl Methoxydibenzoylmethane, Homomenthyl Salicylate, ethylhexyl methoxycinnamate, Benzophenone-3, Ethylhexyl dimethyl para-aminobenzoic acid, Benzophenone-4, Trolamine Salicylate, Terephthalylidene Dicamphor Sulfonic Acid, Drometrizole Trisiloxane and 4-Methylbenzilidene Camphor polysilicone-16. Avobenzone, Diethylhexyl Butamido Triazone, Tris-biphenyl triazine, Tris-biphenyltrazine, Oxybenzone, Octyltriazone, Octisalate, Padimate O, Bis-piperazine HAA299, (2-Ethoxyethyl (2Z)-2-cyano-2-[3-(3-methoxypropylamino) cyclohex-2-en-1-ylidene]acetate), Sulisobenzone, Drometrizole trisiloxane, Methylene bis-benzotriazolyl tetramethylbutylphenol, Benzylidene camphor sulfonic acid, 4-Methylbenzylidene Camphor, Polysilicone-15, Sulisobenzone sodium, Aminobenzoic acid, PEG-25 PABA, Menthyl anthranilate, Isopentyl-4-methoxycinnamate, Dioxibenzone, Cinoxate, camphor benzalkonium sulfate and mixtures thereof.

While the inorganic compound is selected among Zinc Oxide, Titanium Dioxide, Aluminum Stearate, Alumina, Hydrated Silica or Aluminum Hydroxide, their mixtures, and surface modified oxides.

In a specific formulation, the UV filters can also include bacterial based systems.

In a preferred embodiment, the SPF booster particles are characterized by a crystalline structure with a crystallinity higher than 30% and an aspect ratio comprised between 0.1 and 1, where the aspect ratio is defined as the ratio between the width (W) and height (H) based on the bounding rectangle projection of the particle's shape in a 2D image.

According to a further aspect of the invention, said booster particles are characterized by a refractive index comprised between 1.35 and 1.65.

Both inorganic and organic materials can be employed as SPF booster characterized by the specific dimension distribution reported above.

Specifically, preferred inorganic particles are zeolites characterized by an orthorhombic or cubic cell structure and a surface area comprised between 200 and 1000 m$^2$/g.

In a preferred embodiment said zeolites are selected among Faujasite type (FAU), Linde type A (LTA), Mordenite type (MOR), Beta type or a mixture of Linde type A (LTA) and ZSM-5 type.

While, preferred organic particles according to the invention are metal organic frameworks, preferably cyclodextrins, with a surface area comprised between 1000 and 5000 m$^2$/g.

As reported in the below experimental part, the boost of the UVB performance ensured by said specific zeolites to the final composition is at least 20% and, at the same time, the UVA-PF boosting is at least 10%.

The sunscreen composition herein disclosed can be formulated as a one phase formulation which brings the advantages for the cosmetic aspect since it does not leave a greasy or white cast on skin, it is well suited to use under and over makeup and it does not clog skin pores. But it also ensures benefits for the sunscreen activity since it specifically targets certain areas that need increased protection like cheeks, nose or ears and it is typically the most water-resistant formula.

Thus, the single-phase formulation, preferably in the form of a stick, according to the present invention is a water-free phase selected in a group consisting of all types of oils, waxes, emollients, emulsifiers, silicones, polymers, butters, film formers, solubilizers, organic/inorganic dispersants and rheology modifiers, fats, solvents and carriers, stabilizers, organic and inorganic gellifying ingredients.

In a further embodiment, the same water free phases above cited, can be combined with a water phase in order to have an oil-in water formulation which is characterized by a high sensory and feeling on skin, due to a correct balance between the water and oil phases. Said homogenous oil-in-water emulsions are preferably formulated as a serum or cream.

The sunscreen composition according to the invention can also comprise at least one of a cosmetically acceptable emollient, carrier fluid, pigment, humectant, vitamin, antioxidant, natural colorant, preservatives, aroma ingredient, conditioner, emulsifiers, solubilizer, dispersant, film former, rheology modifier, sensory modifier, solvent, carrier, stabilizer, texturizer, filler, extract, essential oil or surfactant.

The present invention also refers to the composition according to any of the embodiment herein disclosed as a sunscreen composition or agent. In particular, the invention refers to a composition according to any of the embodiment herein disclosed for use in a method for protecting a keratinous surface (e.g. skin) from UV radiation comprising contacting the keratinous surface with the sunscreen composition of the invention.

The invention also refers to the composition as herein disclosed as an additive or adjuvant to a sunscreen composition.

Surprisingly, the SPF boosting effect is reached by the compositions and/or functionalized zeolites of the invention without the zeolite binding to the filter, e.g., with no absorption or encapsulation. According to the present invention, in fact, the zeolite and the UV filter do not interact but simply form a physical mixture. As demonstrated by SEM images (FESEM - EDS (Supra 55VP / Zeiss + UltraMax 170mm2 / Oxford Instruments)), according to the present invention, the zeolite is not integrated and is not part of the UV filter and does not act as a carrier. As will be apparent from the following experimental section, the zeolites according to the invention have rather a booster action. This is also confirmed by the fact that without a UV filter, zeolites of the invention have no action as a solar filter.

As will be apparent to a person skilled in the art, the composition of the invention can be included in diverse items and products such as: leave-on skin lotions and creams, shampoos, hair conditioners, shower gels, toilette bars, antiperspirants, deodorants, shave creams, lipsticks, lip-balms, stick formulations, foundations, sunscreen lotions/creams and the like. It is noted that sunscreen agents are active only when exposed to UV radiations. In cases where no prevention against UV radiations/sunburn is needed, for example when the composition is used as a shower gel, applying the composition to the skin cannot be considered as a therapeutic treatment and the composition is mostly used as a cosmetic rather than a medicament.

Therefore, the present invention also refers to non-therapeutic uses, e.g., cosmetic uses, of the composition, according to any one of the embodiments disclosed herein.

Other aspects and advantages of the invention will be apparent from the experimental section below.

## EXAMPLES

Hereinafter, the invention will be explained in more detail with reference to the following non-limiting examples. Modifications or variations of the embodiments here exemplified, obvious to an expert in the art, are encompassed by the appended claims.

Preparation of comparative and inventive sunscreen compositions (P5, S1-S2)

Reference sunscreen formulation P5 as stated in ISO norm 24444:2019 "Cosmetics - Sun protection test Methods", is used as base formulation for SPF booster and UVA-PF booster evaluation. As reported in Table 1 sunscreen reference formulation P5 contains a mixture of different chemical filters made of 3 %wt of butyl methoxydibenzoylmethane, 10%wt of octocrylene, 5%wt ethylhexyl salicylate, 5%wt of benzophenone-3.

Table 1. P5 reference formulation

| Ingredients | % of total mass |
|---|---|
| Phase A1 | |
| Water | 39,35 |
| Disodium EDTA | 0,05 |
| Methylparaben | 0,35 |

(continued)

| Ingredients | % of total mass |
|---|---|
| Phase A1 | |
| Chlorphenesin | 0,2 |
| Phenoxyethanol | 0,7 |
| Phase A2 | |
| Glycerin | 5 |
| Phase B1 | |
| Xanthan Gum | 0,01 |
| Butyl Methoxydibenzoylmethane | 3 |
| Octocrylene | 10 |
| ethylhexyl Salicylate | 5 |
| Benzophenone-3 | 5 |
| Phase B2 | |
| PPG-2 Myristyl Ether Propionate | 2 |
| Octyldodecyl Neopentanoate | 2 |
| Butyloctyl Salicylate | 8 |
| PVP/Eicosene Copolymer | 1,3 |
| Phase B3 | |
| Polyglyceryl-3 Methyl Glucose Distearate | 2 |
| Cetyl Alcohol | 0,5 |
| Stearic Acid | 1 |
| Butylparaben | 0,03 |
| Phase C | |
| Cyclopentasiloxane | 3 |
| Acrylates/C10-30 Alkyl Acrylates Crosspolymer | 0,2 |
| Phase D | |
| Water | 1 |
| Triethanolamine (99 %) | 0,06 |
| Phase E | |
| Water | 10 |
| Potassium Cetyl Phosphate | 0,25 |

The final formulation of S1 and S2 is then prepared by transferring 97g of P5 in a mixer jar and adding 3,0 grams of "SPF booster" under stirring. Then homogenizer is used to allow a fine particles distribution.

Preparation of Stick Formulation-UV filters - Formulation Placebo #B1

Methodology:

1. Combine and mix together the reagents of phase A (1-5) and heat up at 80-85°C, until well melted and homogenous.
2. Separately, blend together all the reagents of phase B (6-9) and heat up at 80-85°C, until well melted and homogenous.
3. Add phase B to phase A and stir until homogenous.

4. Take it off the heat and mix with prior-blended phase C (10-12). Pour off while still molten.

Table 2.

| Phase | Reagent | INCI | w/w% | Function |
|---|---|---|---|---|
| A. | | | | |
| 1. | Caprylic/capric triglicerides | Caprylic/capric triglicerides | 5 | Emolient |
| 2. | Isoamyl laurate | Isoamyl laurate | 13 | Emolient |
| 3. | C12-C15 Alkyl benzoate | C12-C15 Alkyl benzoate | 8 | Emolient |
| 4. | Oilkemia™ 5S polymer, Lubrizol | Caprylic/capric triglicerides (and) polyurethane-79 | 40 | Dispersant / Rheology Modifier / Sensory Modifier / Suspending Agent / Thickener |
| 5. | SPF Booster | | 0 | SPF Booster |
| B. | | | | |
| 7. | Parsol 340®, DSM | Octocrylene | 10 | UV filter |
| 8. | Parsol EHS®, DSM | Ethylhexyl Salicylate | 5 | UV filter |
| 9. | Parsol HMS®, DSM | Homosalate | 10 | UV filter |
| | Parsol 1789®, DSM | Avobenzone | 3 | UV filter |
| C. | | | | |
| 10. | Carrot oil | Zea Mays Germ Oil, Daucus carota sativa Root Extract | 4 | Emolient, colorant |
| 11. | Glucate DO™ emulsifier, Lubrizol | Methyl Glucose Dioleate | 1 | Emollient / Emulsifier |
| 12. | Fragrance | | 1 | Parfum |

Preparation of Stick Formulation-UV filters and zeolite SPF Booster powder- Formulation samples #S3-S4

Methodology:

1. Combine and mix together the reagents of phase A (1-5) and heat up at 80-85°C, until well melted and homogenous.

2. Separately, blend together all the reagents of phase B (6-9) and heat up at 80-85°C, until well melted and homogenous.

3. Add phase B to phase A and stir until homogenous.

4. Take it off the heat and mix with prior-blended phase C (10-12).

Pour off while still molten.

Table 3.

| Phase | Reagent | INCI | w/w% S3 | w/w% S4 | Function |
|---|---|---|---|---|---|
| A. | | | | | |
| 1. | Caprylic/capric triglicerides | Caprylic/capric triglicerides | 5 | 5 | Emolient |
| 2. | Isoamyl laurate | Isoamyl laurate | 13 | 13 | Emolient |

(continued)

| Phase | Reagent | INCI | w/w% S3 | w/w% S4 | Function |
|---|---|---|---|---|---|
| A. | | | | | |
| 3. | C12-C15 Alkyl benzoate | C12-C15 Alkyl benzoate | 8 | 8 | Emolient |
| 4. | Oilkemia™ 5S polymer, Lubrizol | Caprylic/capric triglicerides (and) polyurethane-79 | 39 | 39 | Dispersant / Rheology Modifier / Sensory Modifier / Suspending Agent / Thickener |
| 5. | MOR zeolite | | 3 | - | SPF Booster |
| | BETA zeolite | | - | 3 | |
| B. | | | | | |
| 7. | Parsol 340°, DSM | Octocrylene | 10 | 10 | UV filter |
| 8. | Parsol EHS®, DSM | Ethylhexyl Salicylate | 5 | 5 | UV filter |
| 9. | Parsol HMS®, DSM | Homosalate | 10 | 10 | UV filter |
| | Parsol 1789®, DSM | Avobenzone | 3 | 3 | UV filter |
| C. | | | | | |
| 10. | Carrot oil | Zea Mays Germ Oil, Daucus carota sativa Root Extract | 4 | 4 | Emolient, colorant |
| 11. | Glucate DO™ emulsifier, Lubrizol | Methyl Glucose Dioleate | 1 | 1 | Emollient / Emulsifier |
| 12. | Fragrance | | 0,5 | 0,5 | Parfum |

Preparation of Oil in Water cream Formulation-UV filters and zeolite SPF Booster powder- Placebo B2 and sample #S5

Zeolites were incorporated into a ready, marine-friendly solar emulsion through post-process addition. Specifically, a ready O/W emulsion with SPF 50 was mixed with BETA zeolites using a Turrax at 5000 r.p.m for 5 minutes at room temperature until homogenously blended.

Table 4

| Phase | INCI | % W/W P7 | %W/W S5 |
|---|---|---|---|
| Continueous phase | Aqua (Water) | 100 | 97 |
| UV filters | Diethylamino Hydroxybenzoyl Hexyl Benzoate-DHHB, | | |
| | Ethylhexyl Salicylate- EHS | | |
| | Ethylhexyl Triazone- EHT | | |
| | Bis-Ethylhexyloxyphenol Methoxyphenyl Triazine-BEMT | | |
| | Phenylbenzimidazole Sulfonic Acid-PBSA | | |
| Emulsifier | Polyglyceryl-6 Stearate | | |

(continued)

| Phase | INCI | % W/W P7 | %W/W S5 |
|---|---|---|---|
| Other re-agents | C12-15 Alkyl Benzoate, Butyloctyl Salicylate, Lactobacillus Ferment, Polyglyceryl-6 Stearate, Vp/Eicosene Copolymer, Methyl Glucose Sesquistearate, Potassium Cetyl Phosphate, Glycerin, Aminomethyl Propanol, Hydroxyacetophenone, Ethylhexylglycerin, Polyglyceryl-6 Behenate, Tocopheryl Acetate, Sodium Gluconate, O-Cymen-5-Ol, Diglycerin, Pinus Pinaster Extract (Pinus Pinaster Bark Extract), Sodium Hydroxide, Ethylhexylglycerin, Hydroxyacetophenone, O-Cymen-5-Ol | | |
| SPF Booster | BETA 23.8/NH4+ | - | - |
| | BETA 38/ NH4+ | - | - |
| | BETA307/H+ | - | 3 |

**In Vitro UVA Protection Factor and Critical Wavelength according to ISO 24443:2021 international standard (for S1-S2, C1-C2 and P5)**

The test is based on the assessment of UV-transmittance brought by a thin film of a sunscreen product spread on a roughened substrate by means of a spectrophotometric method. The Helioplate SB6 Sandblasted PMMA plate from HelioScreen Cosmetic Science SAS with the batch number 498 has been used. Standard quantity of 1,2 mg/cm$^2$ ($\pm$1.5%) of cosmetic formulation has been applied onto each substrate by using an automatic dispenser. The minimum application area and size was at least equal to 22,1 cm$^2$. Finally, the applied sunscreen product was submitted to a drying step for least 30 min and less than 60 min in the dark conditions.

Measurements were performed before and after UV exposure with a specific measured and controlled dose of UV radiation from a defined UV exposure source to take into account the potential photo-stability characteristics of the test product. For this test a pre-irradiation dose of 25 J/cm2 was applied.

**In Vitro SPF (Sun Protection Factor) according to ISO/DIS 23675 (for S1-S2, C1-C2 and P5)**

The test is based on the assessment of the UVB (Ultraviolet-B) protection of sunscreen products by means of a UV spectral absorbance curve from an in vitro procedure recommended by Cosmetics Europe [Annex I: Double Plate Method Protocol - COSMETICS EUROPE RECOMMENDATION N° 26 ON THE USE OF ALTERNATIVE METHODS TO ISO24444:2019] and under progress at the ISO level under the project ISO/DIS 23675[ISO/DIS 23675 - Cosmetics - Sun protection test Methods - In Vitro determination of Sun Protection Factor]. Results of this measurement procedure are used for calculation of Sun Protection Factor (SPF).

**SPF in-vitro evaluation (for S3-S7 and B1-B2)**

It has been developed an internal *full in-vitro* method to calculate the protection factor (SPF in vitro) of a sunprotection product against erythema-inducing radiation calculated with a calibration based on a set of sunprotection standards.

The method is formulated according to the principles recommended (Protocol named "In vitro SPF Double Plate method) by the European Cosmetics and Perfumery association (former Colipa, then Cosmetics Europe) in 2011 and internally modified.

The test is based on the assessment of UV-transmittance through a thin film of sunscreen sample spread on a roughened PMMA (Poly(methyl-methacrylate)) substrate. In the inspiring procedure, exposure to a radiation from an UV exposure source is required. UV exposure is not used in this method; each set of sunscreen transmission data is mathematically corrected so that the in vitro SPF data yield the same SPF values provided by several certified SPF standards.

A Perkin-Elmer LAMBDA 1050+ UV/Vis/NIR spectrophotometer equipped with a 150 mm Perkin-Elmer LAMBDA integrating sphere module is used to measure the absorbance A properties of the sunscreen on the test plates and absorbance values are used to calculate SPF through the following integral formula:

$$SPF_{in\ vitro} = \frac{\int_{290\ nm}^{400\ nm} E(\lambda) \times I(\lambda) d\lambda}{\int_{290\ nm}^{400\ nm} E(\lambda) \times I(\lambda) \times 10^{-C \times A(\lambda)} d\lambda}$$

Where:

E: tabled CIE-1987 erythema action spectrum;
I: tabled Midday mid-summer global irradiance at 40°N;
A: measured absorbance;
C: correction coefficient

The UV spectrophotometer wavelength range shall span the primary waveband of 290 to 400 nm, covering both the ranges 290-320 nm (UV-B) and 320-400 nm (UV-A) required in SPF calculation.

A correction coefficient C such that $SPF_{in\ vitro} = SPF_{i,\ standard}$, i=P1, P2, P3 (certified standards in different ranges of sunprotection, see table herebelow for certified values) is used in the calculation.

| Mean and Acceptance limits of SPF reference formulations -Compliance ISO 24444:2019 | | | |
|---|---|---|---|
| formulation | mean | Lower Limit | Upper Limit |
| P2 | 16.1 | 13.7 | 18.5 |
| P5 | 30.6 | 23.7 | 37.4 |
| P8 | 63.1 | 43.9 | 82.3 |

In the following table it is reported a mean SPF boosting value (%), calculated as:

$$mean\ SPF\ boosting\ (\%) = \frac{[(SPF_B - SPF_P) - (SPF_F - SPF_P)]}{SPF_F - SPF_P} \times 100$$

Where:

$SPF_B$: measured SPF of sample formulation with UV filter plus UV booster;

$SPF_F$: measured SPF of sample formulation with UV filter;

$SPF_P$: measured SPF of placebo formulation (without UV filter and UV booster)

Table 5

| Ref. | SPF booster | Framework | Fractions ratio | SPF | SPF boosting (%) | UVA-PF | UVA-PF boosting (%) |
|---|---|---|---|---|---|---|---|
| P5 | no | n.a. | n.a. | 39,1 | 0 | 15,1 | 0 |
| B1 | no | n.a. | n.a. | 26,0 | 0 | 11,0 | 0 |
| B2 | no | n.a. | n.a. | 59,7 | 0 | 24,0 | 0 |
| S1 | Zeolite | LTA + ZSM-5 | 0,9 | 53,7 | 37 | 20,9 | 38 |
| S2 | Zeolite | FAU | 2,1 | 48,3 | 24 | 17,0 | 13 |
| S3 | Zeolite | MOR | 0,5 | 56,2 | 113 | 20,1 | 83 |
| S4 | Zeolite | BETA | 1,6 | 52,6 | 99 | 18,3 | 66 |
| S5 | Zeolite | BETA | 1,6 | 136,1 | 128 | 44,6 | 86 |
| S6 | Zeolite | LTA | 1,4 | 93,1 | 56 | 35,0 | 46 |
| S7 | Zeolite | LTA | 1,0 | 101,3 | 70 | 31,0 | 29 |

(continued)

| Ref. | SPF booster | Framework | Fractions ratio | SPF | SPF boosting (%) | UVA-PF | UVA-PF boosting (%) |
|------|-------------|-----------|-----------------|-----|------------------|--------|---------------------|
| C1 | Zeolite | ZSM-5 | 5,1 | 45,6 | 16 | 16,2 | 7 |
| C2 | silica | n.a. | 0,01 | 50,9 | 30 | 16,5 | 9 |

Table 5, clearly shows that the presence of an SPF booster based on microporous particles having the specific particle size distribution (PSD) of the present invention contribute to a boost both the UVB and the UVA performances.

Indeed, when it is employed an SPF booster characterized by a ratio between the second fraction and the sum of the first and third fractions comprised between 0.1 and 5.0 (see samples S1-S7) the SPF booster is higher than 20% and, at the same time, the UVA-PF boosting is increase of at least 10%.

On the contrary, zeolite particles with a higher ratio (see C1) do not reach the required increased of both UVB and UVA values. At the same time, typical UV boosters such as the silica particles reported in counterexample C2 characterized by a ratio of 0.01, show that even if they are characterized by an SPF boosting effect of at least 30%, they are not able to significantly boost the UVA-PF values.

**SPF in-vivo evaluation**

As a further confirmation of the effectiveness of the present invention, the formulation according to the present invention have also been tested in vivo to assess the SPF of sunscreen formulations according to ISO 24444:2019 standard method. Ten healthy male and females subjects having skin phototype I to III according Fitzpatrick are enrolled by a board certified dermatologist.

A section of each subject's skin is exposed to ultraviolet light without any protection and another (different) section is exposed after application of the sunscreen product to be tested. One further section is exposed after application of a SPF reference sunscreen formulation (used for validation). To determine the SPF, incremental series of delayed erythemal responses are induced on a number of small sub-sites on the skin. These responses are visually assessed for presence of redness 20±4 hours after UV exposure, by a trained technician.

The minimal erythemal dose (MED) for unprotected skin (MEDu) and the MED obtained after application of a sunscreen product (MEDp) are determined on the same subject on the same day. An individual sun protection factor (SPFi) for each subject tested is calculated as the ratio of individual MED on product protected skin divided by the individual MED on unprotected skin i.e., MEDp/MEDu. The sun protection factor for the product (SPF) is the arithmetic mean of all valid SPFi results from each subject in the test

The test is considered valid for the first ten subjects if the resulting range of the 95 % CI of the mean SPF is within ±17% of the mean SPF. If it is not within ±17 % of the mean SPF, the number of subjects is increased stepwise from the minimum number of 10 until the 95 % CI statistical criterion is met.

Table 6. In vivo results

| Ref. | Ingredient as SPF booster | Formulation | SPF value ± StDev | % SPF boosting |
|------|---------------------------|-------------|-------------------|----------------|
| B1 | none | Stick - water free | 30.1 ± 2.4 | Negligible |
| B2 | none | Serum - oil in water | 18.5 ± 3.1 | Negligible |
| S8 | FAU zeolite | Stick - water free | 37.2± 4.1 | 25.54 |

**Claims**

1. A sunscreen composition comprising:

> a) at least one UV filter; and
> b) at least an SPF booster based on microporous particles having a particle size distribution (PSD) comprised between 100 nm and 10 $\mu$m, said PSD including

>> - a first fraction from 100nm to 400nm,
>> - a second fraction from 400nm to 1 $\mu$m, and
>> - a third fraction from 1 to 10 $\mu$m

**characterized in that**, the ratio between the amount of the second fraction and the sum of first and third fractions is comprised between 0.1 and 5.

2. The composition according to claim 1, wherein the amount of SPF booster particles is comprised between 0.1 and 5% wt with respect to the total weight of the composition.

3. The composition according to claim 1 or 2, wherein the amount of the UV filter is comprised 1.0 and 35%wt with respect to the total weight of the composition.

4. The composition according to any of the previous claims, wherein the SPF booster particles are **characterized by** a crystalline structure with a crystallinity higher than 30%.

5. The composition according to any of the previous claims, wherein the SPF booster particles are **characterized by** an aspect ratio comprised between 0.1 and 1.

6. The composition according to any of the previous claims, wherein the SPF booster particles are **characterized by** a refractive index comprised between 1.35 and 1.65.

7. The composition according to any of the previous claims, wherein the SPF booster particles are made of an inorganic material with a surface area comprised between 200 and 1000 $m^2/g$.

8. The composition according to claim 7, wherein the inorganic particles are zeolites **characterized by** orthorhombic or cubic cell structure.

9. The composition according to claim 8, wherein the zeolites are selected among Faujasite type (FAU), Linde type A (LTA), Mordenite type (MOR), Beta type or a mixture of Linde type A (LTA) and ZSM-5 type zeolites.

10. The composition according to claim 1 to 5, wherein the SPF booster particles are made of an organic material with a surface area comprised between 1000 and 5000 $m^2/g$.

11. The composition according to claim 10, wherein the organic particles are metal organic frameworks, preferably cyclodextrins.

12. The composition according to any of the previous claims, wherein the UV filter is an organic molecule selected in a group consisting of Diethylamino Hydroxybenzoyl Hexyl Benzoate, Ethylhexyl Salicylate, Bis-Ethylhexyloxyphenol Methoxyphenyl Triazine, Ethylhexyl Triazone, Phenylbenzimidazole Sulfonic Acid, Octocrylene, Homosalate, Butyl Methoxydibenzoylmethane, Homomenthyl Salicylate, ethylhexyl methoxycinnamate, Benzophenone-3, Ethylhexyl dimethyl para-aminobenzoic acid, Benzophenone-4, Trolamine Salicylate, Terephthalylidene Dicamphor Sulfonic Acid, Drometrizole Trisiloxane and 4-Methylbenzilidene Camphor polysilicone-16, Avobenzone, Diethylhexyl Butamido Triazone, Tris-biphenyl triazine, Tris-biphenyltrazine, Oxybenzone, Octyltriazone, Octisalate, Padimate O, Bis- piperazine HAA299, (2-Ethoxyethyl (2Z)-2-cyano-2-[3-(3-methoxypropylamino) cyclohex-2-en-1-ylidene]acetate) , Sulisobenzone, Drometrizole trisiloxane, Methylene bis-benzotriazolyl tetramethylbutylphenol, Benzylidene camphor sulfonic acid, 4-Methylbenzylidene Camphor, Polysilicone-15, Sulisobenzone sodium, Aminobenzoic acid, PEG-25 PABA, Menthyl anthranilate, Isopentyl-4-methoxycinnamate, Dioxibenzone, Cinoxate, camphor benzalkonium sulfate or mixtures thereof.

13. The composition according to any of the previous claims, wherein the UV filter is an inorganic compound selected in a group consisting of Zinc Oxide, Titanium Dioxide, Aluminum Stearate, Alumina, Hydrated Silica and Aluminum Hydroxide, their mixtures, and surface modified oxides.

14. The composition according to any of the previous claims, wherein the zeolites provide an SPF-boosting action to the sunscreen composition of at least 20%.

15. The composition according to any of the previous claims, further comprising a water-free phase selected in a group consisting of oils, waxes, emollients, emulsifiers, silicones, polymers, butters, film formers, solubilizers, organic and inorganic dispersants, rheology modifiers, fats, solvents and carriers, stabilizers, organic and inorganic jellifying ingredients.

**16.** The composition according to claim 15 formulated as a stick, an oil or a gel.

**17.** The composition according to claims 1 to 15, wherein the composition is a homogenous oil-in-water emulsion, preferably in form of serum or cream.

**18.** The composition according to any of the previous claims, further comprising at least one of a cosmetically acceptable emollient, carrier fluid, pigment, humectant, vitamin, antioxidant, emulsifier, co-emulsifier, hydrophilic or hydrophobic thickeners, wax, butter, film former, silicone, surfactant, active agent, extract, fragrance, preservative, pH-adjusting agent, suspending agent and chelating agent.

**19.** The composition according to any one of the previous claims for use as a sunscreen composition.

**Patentansprüche**

**1.** Sonnenschutzzusammensetzung, umfassend:

a) mindestens einen UV-Filter; und
b) mindestens einen LSF-Verstärker auf der Basis von mikroporösen Partikeln mit einer Partikelgrößenverteilung (PSD) zwischen 100 nm und 10 $\mu$m, wobei die PSD einschließt

- eine erste Fraktion von 100 nm bis 400 nm,
- eine zweite Fraktion von 400 nm bis 1 $\mu$m, und
- eine dritte Fraktion von 1 bis 10 $\mu$m

**dadurch gekennzeichnet, dass** das Verhältnis zwischen der Menge der zweiten Fraktion und der Summe der ersten und dritten Fraktionen zwischen 0,1 und 5 liegt.

**2.** Zusammensetzung nach Anspruch 1, wobei die Menge an LSF-Verstärkerpartikeln zwischen 0,1 und 5 Gew.-%, bezogen auf das Gesamtgewicht der Zusammensetzung, liegt.

**3.** Zusammensetzung nach Anspruch 1 oder 2, wobei die Menge des UV-Filters zwischen 1,0 und 35 Gew.-%, bezogen auf das Gesamtgewicht der Zusammensetzung, liegt.

**4.** Zusammensetzung nach einem der vorstehenden Ansprüche, wobei die LSF-Verstärkerpartikel **gekennzeichnet sind durch** eine kristalline Struktur mit einer Kristallinität höher als 30 %.

**5.** Zusammensetzung nach einem der vorstehenden Ansprüche, wobei die LSF-Verstärkerpartikel **gekennzeichnet sind durch** ein Seitenverhältnis zwischen 0,1 und 1.

**6.** Zusammensetzung nach einem der vorstehenden Ansprüche, wobei die LSF-Verstärkerpartikel **gekennzeichnet sind durch** einen Brechungsindex zwischen 1,35 und 1,65.

**7.** Zusammensetzung nach einem der vorstehenden Ansprüche, wobei die LSF-Verstärkerpartikel hergestellt sind aus einem anorganischen Material mit einer Oberfläche zwischen 200 und 1000 m$^2$/g.

**8.** Zusammensetzung nach Anspruch 7, wobei die anorganischen Partikel Zeolithe sind, **gekennzeichnet durch** eine orthorhombische oder kubische Zellstruktur.

**9.** Zusammensetzung nach Anspruch 8, wobei die Zeolithe ausgewählt sind Zeolithen vom Faujasit-Typ (FAU), Linde-Typ A (LTA), Mordenit-Typ (MOR), Beta-Typ oder einer Mischung aus Linde-Typ A (LTA) und ZSM-5-Typ.

**10.** Zusammensetzung nach Anspruch 1 bis 5, wobei die LSF-Verstärkerpartikel hergestellt sind aus einem organischen Material mit einer Oberfläche zwischen 1000 und 5000 m$^2$/g.

**11.** Zusammensetzung nach Anspruch 10, wobei die organischen Partikel metallorganische Gerüstverbindungen sind, vorzugsweise Cyclodextrine.

**12.** Zusammensetzung nach einem der vorstehenden Ansprüche, wobei der UV-Filter ein organisches Molekül ist, ausgewählt aus einer Gruppe bestehend aus Diethylamino-Hydroxybenzoyl-Hexylbenzoat, Ethylhexylsalicylat, Bis-Ethylhexyloxyphenol-Methoxyphenyltriazin, Ethylhexyltriazon, Phenylbenzimidazolsulfonsäure, Octocrylen, Homosalat, Butylmethoxydibenzoylmethan, Homomenthylsalicylat, Ethylhexylmethoxycinnamat, Benzophenon-3, Ethylhexyldimethyl-p-aminobenzoesäure, Benzophenon-4, Trolaminsalicylat, Terephthalylidendicamphorsulfonsäure, Drometrizol-Trisiloxan und 4-Methylbenziliden-Campher-Polysilikon-16, Avobenzon, Diethylhexylbutamidotriazon, Tris-Biphenyltriazin, Tris-Biphenyltriazin, Oxybenzon, Octyltriazon, Octisalat, Padimat O, Bis-Piperazin HAA299, (2-Ethoxyethyl (2Z)-2-cyano-2-[3-(3-methoxypropylamino) cyclohex-2-en-1-yliden]acetat), Sulisobenzon, Drometrizol-Trisiloxan, Methylenbisbenzotriazolyl-tetramethylbutylphenol, Benzylidenkampfersulfonsäure, 4-Methylbenzylidenkampfer, Polysilicone-15, Sulisobenzon-Natrium, Aminobenzoesäure, PEG-25 PABA, Menthylanthranilat, Isopentyl-4-methoxycinnamat, Dioxibenzon, Cinoxat, Kampferbenzalkoniumsulfat oder Mischungen davon.

**13.** Zusammensetzung nach einem der vorstehenden Ansprüche, wobei der UV-Filter eine anorganische Verbindung ist, die ausgewählt ist aus einer Gruppe bestehend aus Zinkoxid, Titandioxid, Aluminiumstearat, Aluminiumoxid, hydratisiertem Siliciumdioxid und Aluminiumhydroxid, deren Mischungen und oberflächenmodifizierten Oxiden.

**14.** Zusammensetzung nach einem der vorstehenden Ansprüche, wobei die Zeolithe eine LSF-verstärkende Wirkung auf die Sonnenschutzzusammensetzung von mindestens 20 % bereitstellen.

**15.** Zusammensetzung nach einem der vorstehenden Ansprüche, ferner umfassend eine wasserfreie Phase ausgewählt aus einer Gruppe bestehend aus Ölen, Wachsen, Weichmachern, Emulgatoren, Silikonen, Polymeren, Buttern, Filmbildnern, Solubilisatoren, organischen und anorganischen Dispergiermitteln, Rheologiemodifikatoren, Fetten, Lösungsmitteln und Trägern, Stabilisatoren, organischen und anorganischen Geliermitteln.

**16.** Zusammensetzung nach Anspruch 15, formuliert als Stift, Öl oder Gel.

**17.** Zusammensetzung nach den Ansprüchen 1 bis 15, wobei die Zusammensetzung eine homogene Öl-in-Wasser-Emulsion ist, vorzugsweise in Form eines Serums oder einer Creme.

**18.** Zusammensetzung nach einem der vorstehenden Ansprüche, ferner umfassend mindestens eines von einem kosmetisch verträglichen Weichmacher, Trägerfluid, Pigment, Feuchthaltemittel, Vitamin, Antioxidans, Emulgator, CoEmulgator, hydrophilen oder hydrophoben Verdickungsmitteln, Wachs, Butter, Filmbildner, Silikon, Tensid, Wirkstoff, Extrakt, Duftstoff, Konservierungsmittel, pH-Einstellmittel, Suspensionsmittel und Chelatbildner.

**19.** Zusammensetzung nach einem der vorstehenden Ansprüche zur Verwendung als Sonnenschutzzusammensetzung.

**Revendications**

**1.** Composition de protection solaire comprenant :

a) au moins un filtre UV ; et
b) au moins un booster SPF à base de particules microporeuses ayant une distribution granulométrique (PSD) comprise entre 100 nm et 10 $\mu$m, ladite PSD comportant

- une première fraction allant de 100 nm à 400 nm,
- une deuxième fraction allant de 400 nm à 1 $\mu$m, et
- une troisième fraction de 1 à 10 $\mu$m

**caractérisée en ce que** le rapport entre la quantité de la deuxième fraction et la somme des première et troisième fractions est compris entre 0,1 et 5.

**2.** Composition selon la revendication 1, dans laquelle la quantité de particules de renforcement de SPF est comprise entre 0,1 et 5 % en poids par rapport au poids total de la composition.

**3.** Composition selon la revendication 1 ou 2, dans laquelle la quantité du filtre UV est comprise entre 1,0 et 35 % en poids par rapport au poids total de la composition.

4. Composition selon l'une quelconque des revendications précédentes, dans laquelle les particules de renforcement de SPF sont **caractérisées par** une structure cristalline avec une cristallinité supérieure à 30 %.

5. Composition selon l'une quelconque des revendications précédentes, dans laquelle les particules de renforcement de SPF sont **caractérisées par** un rapport d'aspect compris entre 0,1 et 1.

6. Composition selon l'une quelconque des revendications précédentes, dans laquelle les particules de renforcement SPF sont **caractérisées par** un indice de réfraction compris entre 1,35 et 1,65.

7. Composition selon l'une quelconque des revendications précédentes, dans laquelle les particules de renforcement de SPF sont constituées d'un matériau inorganique dont la surface est comprise entre 200 et 1000 m$^2$/g.

8. Composition selon la revendication 7, dans laquelle les particules inorganiques sont des zéolithes **caractérisées par** une structure cellulaire orthorhombique ou cubique.

9. Composition selon la revendication 8, dans laquelle les zéolithes sont choisies parmi les zéolithes de type faujasite (FAU), de type Linde de type A (LTA), de type mordénite (MOR), de type Beta ou un mélange de zéolithes de type Linde de type A (LTA) et de type ZSM-5.

10. Composition selon la revendication 1 à 5, dans laquelle les particules de renforcement de SPF sont constituées d'un matériau organique dont la surface est comprise entre 1 000 et 5 000 m$^2$/g.

11. Composition selon la revendication 10, dans laquelle les particules organiques sont des structures organiques métalliques, de préférence des cyclodextrines.

12. Composition selon l'une quelconque des revendications précédentes, dans laquelle le filtre UV est une molécule organique choisie dans un groupe constitué de benzoate de diéthylaminohydroxybenzoyle-hexyle, salicylate d'éthylhexyle, bis-éthylhexyloxyphénol méthoxyphényltriazine, triazone d'éthylhexyle, acide phénylbenzimidazole sulfonique, octocrylène, homosalate, butyle méthoxydibenzoylméthane, salicylate d'homomenthyle, méthoxycinnamate d'éthylhexyle, benzophénone-3, acide éthylhexylhexyle-diméthyl para-aminobenzoïque, benzophénone-4, salicylate de trolamine, acide téréphtalilidène dicamphor sulfonique, drométrizole trisiloxane et 4-méthylbenzilidène camphre polysilicone-16, avobenzone, diéthylhexyl butamido triazone, tris-biphényl triazine, tris-biphényltrazine, oxybenzone, octyltriazone, octisalate, padimate O, bis-pipérazine HAA299, (2-éthoxyéthyl (2Z)-2-cyano-2-[3-(3-méthoxypropylamino) cyclohex-2-en-1-ylidène]acétate), sulisobenzone, drométrizole trisiloxane, méthylène bis-benzotriazolyl tétraméthylbutylphénol, benzylidène camphre acide sulfonique, camphre 4-méthylbenzylidène, polysilicone-15, sulisobenzone sodique, acide aminobenzoïque, PEG-25 PABA, anthranilate menthyle, Isopentyl-4-méthoxycinnamate, dioxibenzone, cinoxate, camphre Benzalkonium sulfate ou mélanges de ceux-ci.

13. Composition selon l'une quelconque des revendications précédentes, dans laquelle le filtre UV est un composé inorganique choisi dans un groupe constitué d'oxyde de zinc, dioxyde de titane, stéarate d'aluminium, alumine, silice hydratée et hydroxyde d'aluminium, leurs mélanges et des oxydes modifiés en surface.

14. Composition selon l'une quelconque des revendications précédentes, dans laquelle les zéolithes confèrent une action d'amplification de SPF d'au moins 20 % à la composition de protection solaire.

15. Composition selon l'une quelconque des revendications précédentes, comprenant en outre une phase exempte d'eau choisie dans un groupe constitué d'huiles, cires, émollients, émulsifiants, silicones, polymères, beurres, filmogènes, solubilisants, dispersants organiques et inorganiques, rhéologie des modificateurs, des graisses, des solvants et des supports, des stabilisants, des ingrédients gélifiants organiques et inorganiques.

16. Composition selon la revendication 15, formulée sous forme de bâtonnet, d'huile ou de gel.

17. Composition selon les revendications 1 à 15, dans laquelle la composition est une émulsion huile-dans-eau homogène, de préférence sous forme de sérum ou de crème.

18. Composition selon l'une quelconque des revendications précédentes, comprenant en outre au moins l'un parmi un émollient cosmétiquement acceptable, fluide porteur, pigment, humectant, vitamine, antioxydant, émulsifiant, co-émulsifiant, épaississants hydrophiles ou hydrophobes, cire, beurre, agent filmogène, silicone, tensioactif, agent actif, extrait, parfum, conservateur, agent d'ajustement de pH, agent de suspension, agent chélatant.

19. Composition selon l'une quelconque des revendications précédentes pour une utilisation en tant que composition de protection solaire.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2017112982 A
- WO 2021102873 A
- US 9265715 B
- US 20210330571 A
- WO 2022081942 A
- US 10485745 B